# FASCICULE DE BREVET EUROPEEN

(11) **EP 3 261 567 B1**
(45) Date de publication et mention de la délivrance du brevet: **03.07.2019**
(21) Numéro de dépôt: 16707933.4
(22) Date de dépôt: 17.02.2016
(51) Int. Cl.: A61B 17/80, A61B 17/82

(54) **DISPOSITIF PROTHÉTIQUE DE STERNUM AVEC MANUBRIUM**
PROTHETISCHE STERNUMVORRICHTUNG MIT MANUBRIUM
PROSTHETIC STERNUM DEVICE WITH MANUBRIUM

(30) Priorité: 24.02.2015 FR 1551572; 13.01.2016 FR 1650272; 13.01.2016 FR 1650273
(43) Date de publication de la demande: 03.01.2018
(73) Titulaire: Neuro France Implants, 41160 La Ville-Aux-Clercs (FR)
(72) Inventeur: FABRE, Dominique, 92380 Garches (FR); FADEL, Elie, 92160 Antony (FR); MERCIER, Olaf, 92260 Fontenay Aux Roses (FR); MOREAU, Patrice, 41270 Boursay (FR); MUSSOT, Sacha, 75015 Paris (FR); WORNER, Karin, 41360 Epuisay (FR)
(74) Mandataire: Cabinet Bleger-Rhein-Poupon
(86) Numéro de dépôt international: PCT/FR2016/050362
(87) Numéro de publication internationale: WO 2016/135395

(56) Documents cités:
- FR-A1- 3 004 337
- US-A1- 2013 158 608
- US-A1- 2014 257 291

## Description

La présente invention a pour objet un dispositif prothétique de sternum avec manubrium, destiné à être implanté en remplacement d'un sternum et de son manubrium, c'est-à-dire sa partie supérieure sur laquelle s'articulent les clavicules.

On connaît déjà, par le document FR 3 004 337, dispositif prothétique de sternum comportant une plaque et des moyens d'attachement à des côtes, où chacun desdits moyens d'attachement à des côtes comprend une extrémité de solidarisation à ladite plaque et une extrémité munie d'un moyen d'accrochage à une côte, ladite plaque comportant des zones de fixation d'un desdits moyens d'attachement, réparties en périphérie, de part et d'autre d'une zone médiane.

Si un tel dispositif prothétique de sternum constitue un progrès par rapport à l'art antérieur lors du remplacement d'un sternum, il présente toutefois un inconvénient du fait que, lors de l'ablation du sternum, il n'est pas possible de raccorder les clavicules puisque le manubrium a également été enlevé.

La présente invention a pour but de perfectionner le dispositif prothétique de sternum objet du document FR 3 004 337.

Le dispositif prothétique de sternum avec manubrium selon l'invention, comporte une plaque et des moyens d'attachement à des côtes, où chacun desdits moyens d'attachement à des côtes comprend une extrémité de solidarisation à ladite plaque et une extrémité munie d'un moyen d'accrochage à une côte, et il se caractérise en ce que ladite plaque comporte en outre, dans sa région supérieure, deux éléments de solidarisation pour chacun d'une broche, qui consistent chacun en une articulation polyaxiale conformée pour autoriser une mobilité multidirectionnelle de ladite broche.

Selon une caractéristique additionnelle du dispositif prothétique selon l'invention, il comporte en outre deux broches, chacune solidarisée à une articulation polyaxiale, et destinée à fixer une clavicule ou partie de clavicule, prothétique ou non.

Selon une autre caractéristique additionnelle du dispositif prothétique selon l'invention, l'articulation polyaxiale consiste en une rotule.

Selon un mode de réalisation particulier du dispositif prothétique selon l'invention, chaque broche est destinée à être solidarisée à la plaque par une extrémité à un élément de l'articulation polyaxiale.

Selon un autre mode de réalisation particulier du dispositif prothétique selon l'invention, chaque broche est destinée à être solidarisée à la plaque par une extrémité, laquelle extrémité consiste en un élément de l'articulation polyaxiale.

Selon une caractéristique additionnelle du dispositif prothétique selon l'invention, chacune des deux articulations polyaxiales, comprend :
- une extension faisant saillie de la plaque, pourvue d'une cavité de forme hémisphérique, d'axe passant par le plan de ladite plaque et faisant un angle avec l'axe longitudinal principal de ladite plaque, en sorte que ladite cavité s'ouvre vers l'extérieur, le haut et latéralement,
- une tête sphérique destinée à loger dans ladite cavité,
- un couvercle solidarisable à ladite extension, destiné à fermer ladite cavité en enfermant ladite tête sphérique, et comportant une ouverture permettant la liaison d'une broche à ladite tête sphérique.

Selon une caractéristique additionnelle du dispositif prothétique selon l'invention, chaque extension comporte un logement garni d'un insert qui comporte la cavité.

Selon une caractéristique additionnelle du dispositif prothétique selon l'invention, la paroi extérieure de l'insert est sphérique, concentrique à la cavité, tandis que le logement de l'extension est en partie sphérique, congruent audit insert, en sorte de créer une liaison rotule.

Selon une autre caractéristique additionnelle du dispositif prothétique selon l'invention, l'insert et la tête sphérique sont réalisés en PEEK.

Selon une autre caractéristique additionnelle du dispositif prothétique selon l'invention, chaque broche comporte un clou diaphysaire ainsi qu'une plaquette qui s'étend parallèlement audit clou diaphysaire, en regard de celui-ci et à distance en sorte de pouvoir venir, après insertion dudit clou diaphysaire dans la clavicule, en contact extérieurement à cette clavicule, ladite plaquette étant percée d'au moins un trou permettant le passage d'une vis destinée à être vissée dans la matière osseuse.

Selon une autre caractéristique additionnelle du dispositif prothétique selon l'invention, le clou diaphysaire est constitué de trois tiges parallèles disposées en triangle autour d'un axe central.

Les avantages et les caractéristiques du dispositif prothétique de sternum avec manubrium selon l'invention, ressortiront plus clairement de la description qui suit et qui se rapporte au dessin annexé, lequel en représente des modes de réalisation non limitatifs.

Dans le dessin annexé:
- la figure 1 représente une vue en plan avec éclaté partiel du dispositif prothétique de sternum avec manubrium selon l'invention.
- la figure 2 représente une vue schématique en perspective d'une partie du même dispositif.
- la figure 3 représente une vue en perspective et en éclaté du même dispositif.
- la figure 4 représente une vue schématique en plan d'une variante du dispositif prothétique de sternum avec manubrium selon l'invention.
- la figure 5 représente une vue schématique partielle en plan d'une autre variante du dispositif prothétique de sternum avec manubrium selon l'invention.
- la figure 6 représente une vue en perspective d'une variante d'une broche destinée à coopérer avec le dispositif prothétique de sternum avec manubrium selon l'invention.
- la figure 7 représente une vue en perspective d'un mode de réalisation particulier d'une broche destinée à coopérer avec le dispositif prothétique de sternum avec manubrium selon l'invention.

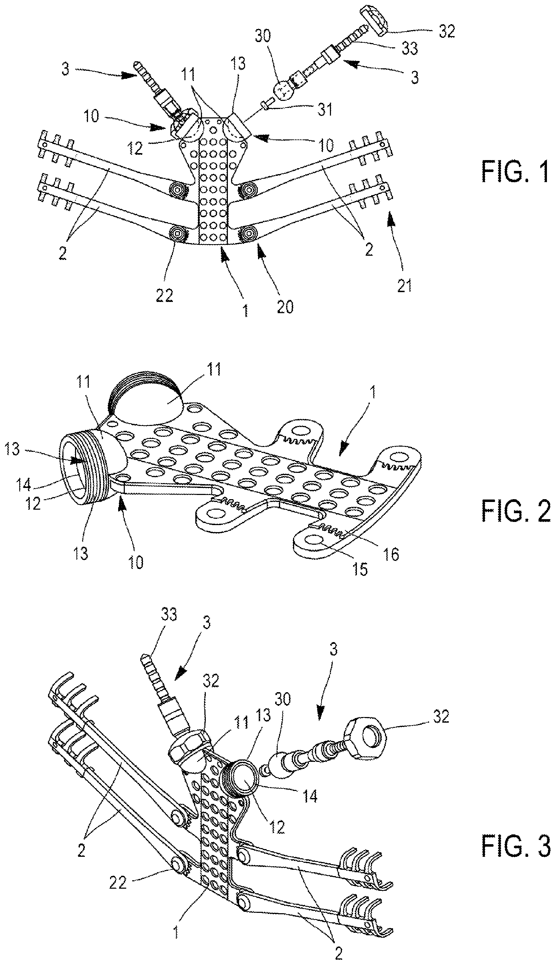

En référence aux figures 1, 2 et 3, on peut voir qu'un dispositif prothétique de sternum avec manubrium selon l'invention comprend, dans ce mode de réalisation, une plaque 1 et quatre moyens 2 d'attachement à une côte, de manière connue en soi conformément au document FR 3 004 337, qui s'étendent latéralement de part et d'autre de la plaque 1.

On notera que conformément au document FR 3 004 337, chacun des moyens d'attachement comporte une extrémité 20 de solidarisation à la plaque 1 et une extrémité 21 munie d'un moyen d'accrochage à une côte. L'extrémité 20 comporte un trou, non visible sur ces figures, traversé par une vis 22 vissée dans l'un des trous taraudés 15 que comporte la plaque 1. Par ailleurs, un moyen d'indexage permet d'immobiliser angulairement chaque moyen d'attachement 2, il est composé d'une part au niveau de chacun des trous taraudés d'une crémaillère 16 disposée en un arc de cercle concentrique au trou taraudé 15, et d'autre part d'un doigt 23 que comporte l'extrémité 20 de chacun des moyens d'attachement 2 s'étendant radialement au trou de passage de la vis 22, et destiné à coopérer avec l'un des crans de la crémaillère 16.

Selon l'invention, la plaque 1 comporte de plus dans sa région supérieure, c'est-à-dire dans la partie extrême opposée à la partie qui porte les moyens d'attachement 2, deux emplacements 10 d'insertion sur chacun d'une broche 3 destinée à être connectée, par insertion d'un clou diaphysaire 33 dans une clavicule, non représentée, après résection, laquelle clavicule peut également être prothétique.

Chaque emplacement 10 se présente sous la forme d'une extension 11 pourvue d'une cavité 12 de forme hémisphérique, d'axe passant par le plan de la plaque 1 et faisant un angle avec l'axe longitudinal principal de la plaque 1, en sorte de s'ouvrir vers l'extérieur, le haut et latéralement. L'extension est munie sur son bord distal et périphériquement d'un filetage 13.

La cavité 12 est destiné à recevoir une tête sphérique 30 qui y est maintenue par un écrou 32 vissé sur le filetage 13, l'ensemble formant une articulation polyaxiale de type rotule.

La broche 3 est destinée à être solidarisée à l'articulation polyaxiale, et à cet effet son extrémité, destinée à être solidarisée à la plaque 1, et la tête sphérique 30 sont assemblées et maintenues par une vis 31.

Dans le mode de réalisation représenté, la broche 3 est solidarisée à l'articulation polyaxiale que constituent l'extension 11, la cavité sphérique 12, l'écrou 32 et la tête sphérique 30, mais il est tout à fait envisageable que la tête sphérique 30 fasse partie intégrante de la broche 3, et par que conséquent la broche 3 comporte une partie de l'articulation polyaxiale.

Il est à noter de manière préférentielle, chaque extension 11 est garnie intérieurement d'un insert 14, qui comporte la cavité 12. De manière avantageuse, la paroi extérieure de l'insert 14 est sphérique, et le logement, non visible, de ce dernier réalisé dans l'extension 11, lui est congruent, en sorte de créer une liaison rotule supplémentaire entre l'insert 14 et l'extension 11.

On notera également que l'insert 14 ainsi que la rotule sphérique 30 sont réalisés, non limitativement en PEEK (polyétheréthercétone).

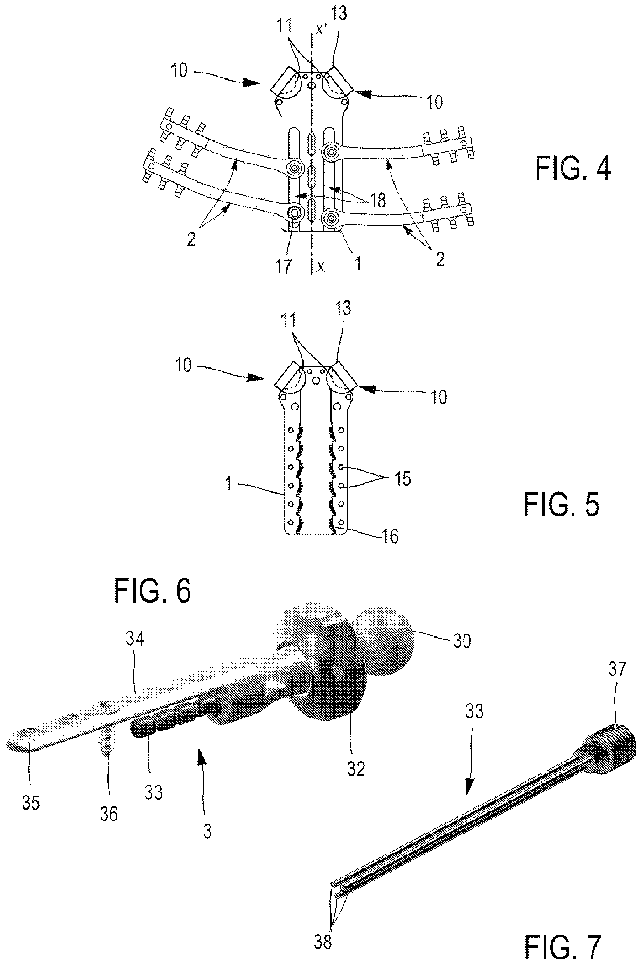

En référence maintenant à la figure 4, on peut voir une variante du dispositif prothétique selon l'invention qui diffère de celui représenté sur les figures 1, 2 et 3, en ce que la solidarisation à la plaque 1 des moyens d'attachement 2 est réalisé au travers d'éléments de glissement 17 à chacun desquels est solidarisé un moyen d'attachement 2, et aptes à se déplacer et à être bloqués dans l'une des deux coulisses 18 que comporte la plaque 1, et disposées longitudinalement de part et d'autre d'un axe longitudinal XX'.

L'association du réglage de la position des moyens d'attachement 2 sur la plaque 1 et de l'orientation multidirectionnelle des broches 3, permet de trouver un positionnement très précis du dispositif prothétique de sternum avec manubrium selon l'invention.

En référence maintenant à la figure 4, on peut voir une variante du dispositif prothétique selon l'invention, où la plaque 1 comporte un nombre de trous taraudés 15 de fixation de moyens d'attachement 2, supérieur à celui de moyens d'attachement 2 à solidariser, en sorte de permettre de choisir les trous taraudés 15 les plus adaptés à l'anatomie du patient.

En référence à la figure 6, on peut voir une broche 3 comprenant un clou diaphysaire 33, une tête sphérique 30 et un écrou 32, ainsi qu'une plaquette 34 qui s'étend parallèlement au clou diaphysaire 33 en regard de celui-ci et à une certaine distance en sorte de pouvoir venir, après insertion du clou diaphysaire 33 dans la clavicule, en contact extérieurement à cette clavicule. La plaquette 34 est percée de trous 35 permettant le passage de vis 36, dont une seule est représentée, destinées à être vissées dans la matière osseuse.

En référence à la figure 7, on peut voir une partie 36 d'une variante d'une broche 3, qui comporte un fût fileté 37 destiné à être vissé dans une tête sphérique non représentée, prolongé de trois tiges parallèles 38 réparties en triangle autour d'un axe central, et destinées à être insérées dans une clavicule, et qui constituent le clou diaphysaire.

## Revendications

1. Dispositif prothétique de sternum avec manubrium, comportant une plaque (1) et des moyens (2) d'attachement à des côtes, où chacun desdits moyens (2) d'attachement à des côtes comprend une extrémité (20) de solidarisation à ladite plaque (1) et une extrémité (21) munie d'un moyen d'accrochage à une côte, **caractérisé en ce que** ladite plaque (1) comporte en outre, dans sa région supérieure, deux éléments (10) de solidarisation pour chacun d'une broche (3), qui consistent chacun en une articulation polyaxiale conformée pour autoriser une mobilité multidirectionnelle de ladite broche (3).

2. Dispositif prothétique de sternum selon la revendication 1, **caractérisé en ce qu'**il comporte en outre deux broches (3), chacune solidarisée à une articulation polyaxiale (10), et destinée à fixer une clavicule ou partie de clavicule, prothétique ou non.

3. Dispositif prothétique de sternum selon la revendication 1 ou la revendication 2, **caractérisé en ce que** l'articulation polyaxiale (10) consiste en une rotule.

4. Dispositif prothétique de sternum selon la revendication 2 ou la revendication 3, **caractérisé en ce que** chaque broche (3) est destinée à être solidarisée à la plaque (1) par une extrémité à un élément (30) de l'articulation polyaxiale (10).

5. Dispositif prothétique de sternum selon la revendication 2 ou la revendication 3,**caractérisé en ce que** chaque broche (3) est destinée à être solidarisée à la plaque (1) par une extrémité, laquelle extrémité consiste en un élément (30) de l'articulation polyaxiale (10).

6. Dispositif prothétique de sternum selon l'une quelconque des revendications 1 à 5, **caractérisé en ce que** chacune des deux articulations polyaxiales (10), comprend :
- une extension (11) faisant saillie de la plaque (1), pourvue d'une cavité (12) de forme hémisphérique, d'axe passant par le plan de ladite plaque (1) et faisant un angle avec l'axe longitudinal principal de ladite plaque (1) en sorte que ladite cavité (12) s'ouvre vers l'extérieur, le haut et latéralement,
- une tête sphérique (30) destinée à loger dans ladite cavité (12),
- un couvercle (32) solidarisable à ladite extension (11), destiné à fermer ladite cavité (12) en enfermant ladite tête sphérique (30), et comportant une ouverture permettant la liaison d'une broche (3) à ladite tête sphérique (30).

7. Dispositif prothétique de sternum selon la revendication 6, **caractérisé en ce que** chaque extension (11) comporte un logement garni d'un insert (14) qui comporte la cavité (12).

8. Dispositif prothétique de sternum selon la revendication 7, **caractérisé en ce que** la paroi extérieure de l'insert (14) est sphérique, concentrique à la cavité (12), tandis que le logement de l'extension (11) est en partie sphérique, congruent audit insert (14), en sorte de créer une liaison rotule.

9. Dispositif prothétique de sternum selon la revendication 7 ou la revendication 8, **caractérisé en ce que** l'insert (14) et la tête sphérique (30) sont réalisés en PEEK.

10. Dispositif prothétique de sternum selon l'une quelconque des revendications 1 à 9, **caractérisé en ce que** la solidarisation à la plaque (1) comporte deux coulisses (18) disposées longitudinalement de part et d'autre d'un axe longitudinal, dans lesquelles sont aptes à se déplacer et à être bloqués d'éléments de glissement (17) à chacun desquels est solidarisé un moyen d'attachement (2).

11. Dispositif prothétique de sternum selon l'une quelconque des revendications 1 à 9, **caractérisé en ce que** la plaque (1) comporte un nombre de trous (15) de fixation de moyens d'attachement (2), supérieur à celui de moyens d'attachement (2) à solidariser.

12. Dispositif prothétique de sternum selon l'une quelconque des revendications 2 à 11, **caractérisé en ce que** chaque broche (3) comporte un clou diaphysaire (33) ainsi qu'une plaquette (34) qui s'étend parallèlement audit clou diaphysaire (33), en regard de celui-ci et à distance en sorte de pouvoir venir, après insertion dudit clou diaphysaire (33) dans la clavicule, en contact extérieurement à cette clavicule, ladite plaquette (34) étant percée d'au moins un trou (35) permettant le passage d'une vis (36) destinée à être vissée dans la matière osseuse.

13. Dispositif prothétique de sternum selon la revendication 12, **caractérisé en ce que** le clou diaphysaire (33) est constitué de trois tiges parallèles (38) disposées en triangle autour d'un axe central.

## Patentansprüche

1. Prothetische Sternumvorrichtung mit Manubrium, aufweisend eine Platte (1) und Verbindungsmittel (2) an Rippen, wobei jedes der Verbindungsmittel (2) an Rippen ein Ende (20) zur festen Befestigung an der Platte (1) und ein Ende (21), das mit einem Haltemittel an einer Rippe versehen ist, umfasst, **dadurch gekennzeichnet, dass** die Platte (1) ferner in ihrem oberen Bereich zwei Elemente (10) zur festen Befestigung für jede einer Spindel (3) aufweist, die jeweils aus einem polyaxialen Gelenk besteht, das ausgebildet ist, um eine multidirektionale Mobilität der Spindel (3) zu gestatten.

2. Prothetische Sternumvorrichtung nach Anspruch 1, **dadurch gekennzeichnet, dass** sie ferner zwei Spindeln (3) aufweist, die jede mit einem polyaxialen Gelenk (10) verbunden und bestimmt sind, ein Schlüsselbein oder Schlüsselbeinteil, prothetisch oder nicht, zu befestigen.

3. Prothetische Sternumvorrichtung nach Anspruch 1 oder Anspruch 2, **dadurch gekennzeichnet, dass** das polyaxiale Gelenk (10) aus einem Kugelgelenk besteht.

4. Prothetische Sternumvorrichtung nach Anspruch 2 oder Anspruch 3, **dadurch gekennzeichnet, dass** jede Spindel (3) bestimmt ist, an der Platte (1) durch ein Ende an einem Element (30) des polyaxialen Gelenks (10) fest befestigt zu sein.

5. Prothetische Sternumvorrichtung nach Anspruch 2 oder Anspruch 3, **dadurch gekennzeichnet, dass** jede Spindel (3) bestimmt ist, an der Platte (1) durch ein Ende fest befestigt zu sein, wobei das Ende aus einem Element (30) des polyaxialen Gelenks (10) besteht.

6. Prothetische Sternumvorrichtung nach einem der Ansprüche 1 bis 5, **dadurch gekennzeichnet, dass** jedes der zwei polyaxialen Gelenke (10) umfasst:
- eine Erweiterung (11), die aus der Platte (1) hervorsteht, versehen mit einem halbkugelig geformten Hohlraum (12), mit einer Achse, die durch die Ebene der Platte (1) verläuft und mit der Hauptlängsachse der Platte (1) derart einen Winkel bildet, dass sich der Hohlraum (12) nach außen, oben und seitlich öffnet,
- einen kugeligen Kopf (30), der bestimmt ist, in dem Hohlraum (12) untergebracht zu sein,
- einen an der Erweiterung (11) fest verbindbaren Deckel (32), der bestimmt ist, den Hohlraum (12) bei Einschluss des kugeligen Kopfes (30) zu verschließen und aufweisend eine Öffnung, die die Verbindung einer Spindel (3) an dem kugeligen Kopf (30) erlaubt.

7. Prothetische Sternumvorrichtung nach Anspruch 6, **dadurch gekennzeichnet, dass** jede Erweiterung (11) eine Aufnahme aufweist, die mit einem Einsatz (14) versehen ist, der den Hohlraum (12) aufweist.

8. Prothetische Sternumvorrichtung nach Anspruch 7, **dadurch gekennzeichnet, dass** die Außenwand des Einsatzes (14) kugelig konzentrisch zum Hohlraum (12) ist, wogegen die Aufnahme der Erweiterung (11) teilweise kugelig kongruent zum Einsatz (14) ist, so dass eine Kugelgelenkverbindung gebildet wird.

9. Prothetische Sternumvorrichtung nach Anspruch 7 oder Anspruch 8, **dadurch gekennzeichnet, dass** der Einsatz (14) und der kugelige Kopf (30) aus PEEK hergestellt sind.

10. Prothetische Sternumvorrichtung nach einem der Ansprüche 1 bis 9, **dadurch gekennzeichnet, dass** die feste Verbindung an der Platte (1) zwei Führungsschienen (18) aufweist, die längs beiderseits einer Längsachse angeordnet sind, in welchen Gleitelemente (17), an denen an jedem ein Verbindungsmittel (2) fest verbunden sind, imstande sind, sich zu verlagern und blockiert zu werden.

11. Prothetische Sternumvorrichtung nach einem der Ansprüche 1 bis 9, **dadurch gekennzeichnet, dass** die Platte (1) eine Anzahl von Befestigungslöchern (15) von Verbindungsmitteln (2) aufweist, die größer ist als die der fest zu verbindenden Verbindungsmittel (2).

12. Prothetische Sternumvorrichtung nach einem der Ansprüche 2 bis 11, **dadurch gekennzeichnet, dass** jede Spindel (3) einen diaphysären Stift (33) sowie ein Plättchen (34) aufweist, das sich parallel zu dem diaphysären Stift (33) gegenüber diesem und derart beabstandet erstreckt, dass es nach dem Einsetzen des diaphysären Stifts (33) in das Schlüsselbei in Außenkontakt mit dem Schlüsselbein kommt, wobei das Plättchen (34) von mindestens einem Loch (35) durchbrochen ist, das den Durchgang einer Schraube (36) erlaubt, die bestimmt ist, in das Knochenmaterial geschraubt zu sein.

13. Prothetische Sternumvorrichtung nach Anspruch 12, **dadurch gekennzeichnet, dass** der diaphysäre Stift (33) aus drei parallelen Stangen (38) besteht, die im Dreieck um eine zentrale Achse angeordnet sind.

## Claims

1. A prosthetic sternum device with manubrium, including a plate (1) and means (2) for attaching to ribs, where each of said means (2) for attaching to ribs comprises an end (20) for securing to said plate (1) and an end (21) provided with a means for attaching to a rib, **characterized in that** said plate (1) further includes, in its upper region, two securing elements (10) each for a pin (3), which each consist of a polyaxial articulation configured to allow multidirectional mobility of said pin (3).

2. The prosthetic sternum device according to claim 1, **characterized in that** it further includes two pins (3), each secured to a polyaxial articulation (10), and intended to fasten a clavicle or clavicle part, which may or may not be prosthetic.

3. The prosthetic sternum device according to claim 1 or claim 2, **characterized in that** the polyaxial articulation (10) consists of a ball and socket joint.

4. The prosthetic sternum device according to claim 2 or claim 3, **characterized in that** each pin (3) is intended to be secured to the plate (1) by one end to an element (30) of the polyaxial articulation (10).

5. The prosthetic sternum device according to claim 2 or claim 3, **characterized in that** each pin (3) is intended to be secured to the plate (1) by one end, said end consisting of an element (30) of the polyaxial articulation (10) .

6. The prosthetic sternum device according to any one of claims 1 to 5, **characterized in that** each of the two polyaxial articulations (10) comprises:
- an extension (11) protruding from the plate (1), provided with a hemispherical cavity (12), with an axis passing through the plane of said plate (1) and forming an angle with the main longitudinal axis of said plate (1), such that said cavity (12) opens outwardly, upwardly and laterally,
- a spherical head (30) intended to be housed in said cavity (12),
- a cover (32) able to be secured to said extension (11), intended to close said cavity (12) by enclosing said spherical head (30), and including an opening allowing a pin (3) to be connected to said spherical head (30).

7. The prosthetic sternum device according to claim 6, **characterized in that** the each extension (11) includes a housing lined with an insert (14) that includes the cavity (12) .

8. The prosthetic sternum device according to claim 7, **characterized in that** the outer wall of the insert (14) is spherical, concentric to the cavity (12), while the housing of the extension (11) is partially spherical, congruent with said insert (14), so as to create a ball and socket connection.

9. The prosthetic sternum device according to claim 7 or claim 8, **characterized in that** the insert (14) and the spherical head (30) are made from PEEK.

10. The prosthetic sternum device according to any one of claims 1 to 9, **characterized in that** the securing to the plate (1) includes two slide channels (18) arranged longitudinally on either side of a longitudinal axis, in which sliding elements (17) can move and be blocked, to each of which an attaching means (2) is secured.

11. The prosthetic sternum device according to any one of claims 1 to 9, **characterized in that** the plate (1) includes a number of attaching means (2) fastening holes (15) greater than the number of attaching means (2) to be secured.

12. The prosthetic sternum device according to any one of claims 2 to 11, **characterized in that** each pin (3) includes a diaphyseal nail (33) as well as a small plate (34) that extends parallel to said diaphyseal nail (33), across and at a distance from the latter so as to be able, after insertion of said diaphyseal nail (33) into the clavicle, to come outwardly into contact with this clavicle, said small plate (34) being pierced with at least one hole (35) allowing the passage of a screw (36) intended to be screwed into the bone.

13. The prosthetic sternum device according to claim 12, **characterized in that** the diaphyseal nail (33) is made up of three parallel rods (38) arranged in a triangle around a central axis.
